# EUROPEAN PATENT SPECIFICATION

(11) **EP 0 613 481 B1**
(45) Date of publication and mention of the grant of the patent: **09.04.1997**
(21) Application number: 92923746.9
(22) Date of filing: 19.11.1992
(51) Int. Cl.: C07F 15/00, A61K 31/28

(54) **TRIAMINO PLATINUM COMPLEXES AND A METHOD FOR THE SYNTHESIS THEREOF**
TRIAMINOPLASTINKOMPLEXE UND VERFAHREN ZUR DEREN HERSTELLUNG
COMPLEXES DE PLATINE TRIAMINO ET LEUR PROCEDE DE SYNTHESE

(30) Priority: 22.11.1991 IT MI91312
(43) Date of publication of application: 07.09.1994
(73) Proprietor: ISTITUTO NAZIONALE PER LA RICERCA SUL CANCRO, I-16132 Genova (IT)
(72) Inventor: ESPOSITO, Mauro, I-16132 Genova (IT)
(74) Representative: Minoja, Fabrizio
(86) International application number: EP9202658
(87) International publication number: WO9310130

(56) References cited:
- EP-A- 0 305 008
- EP-A- 0 409 528
- CHEMICAL ABSTRACTS, vol. 113, 1990, Columbus, Ohio, US; abstract no. 91009a, ESPOSITO, M. ET AL. page 28 ;
- CHEMICAL ABSTRACTS, vol. 117, 1992, Columbus, Ohio, US; abstract no. 62501g, ESPOSITO, M. ET AL. page 48 ;

## Description

The present invention relates to ionic cis-triamino platinum (II) complexes of general formula I

[(aₘ)₂ Pt H₂N-R Cl]⁺ Cl⁻ (HCl)ₙ (H₂O)ₘ (I)

wherein:
aₘ is an amino monodentate ligand selected from the group consisting of NH₃, straight or branched C₁-C₅ alkylamine, straight or branched C₁-C₃ dialkylamine, cyclopropylamine, cyclopentylamine, cyclohexylamine; or both aₘ groups, taken together, from a bidentate amino ligand selected from the group consisting of a C₂-C₈ alkyl-1,2-diamine or a straight or cyclic C₃-C₈ alkyl-1,3-diamine;
R is a group of formula p-NH₂-C₆H₄-CO-X-(CH₂)₂-NRaRb where X is selected from the group consisting of O, NH and CH₂, Ra and Rb, which are the same or different, are each a straight or branched C₁-C₄ alkyl and n and m, independently each other, are 0 or 1.

Further objects of the present invention are a process for the preparation of compounds of formula I and pharmaceutical compositions containing them.

A straight or branched C₁-C₅ alkylamine is preferably propylamine, isopropylamine, n-pentylamine.

A straight or branched C₁-C₃ dialkylamine is preferably diisopropylamine.

A straight C₂-C₈ alkyl-1,2-diamine is preferably 1,2-ethylenediamine or 1,2-butanediamine (1-, d-, dl-or meso).

A cycloaliphatic C₃-C₈ alkyl-1,2-diamine is preferably 1,2-cyclopentanediamine (1-, d-, dl- or meso), 1,2-cyclohexanediamine (1-, d-, dl- or meso) or 1,2-cycloheptanediamine (1-, d-, dl- or meso).

A straight C₃-C₈ alkyl-1,3-diamine is preferably 1,3-propylenediamine.

A cyclic C₃-C₈ alkyl-1,3-diamine is preferably 1,1-bis-aminomethylcyclopentane or 1,1-bis-aminomethyl-cyclohexane.

The most particularly preferred ligand aₘ is the monodentate ligand NH₃; said amino monodentate ligands aₘ are specifically cis-oriented with respect to the Pt atom.

Ra and Rb are preferably both ethyl.

X is preferably O.

When n is the integer 1, the compounds of the invention are addition salts, when m is the integer 1, the compounds of the invention are solvates and contain a molecule of crystallization water.

A particularly preferred compound of the invention is cis-diamino-chloro-4-[diethylammoniumethoxycarbonyl phenyl]amino-platinum (II)⁺ bis-chloride monohydrate.

Antitumor platinum triamine complexes containing aniline or substituted aniline as a ligand are disclosed in EP-A-0409528 (23-1-1991) whereas J. Nat. Cancer Inst., 1990, 82(2), 677-84, discloses mixtures of diamino dichloro platinum with procaine hydrochloride.

The compounds of the invention of formula I are obtained through a process consisting of the following steps:
a) reaction of a cis-diamino dichloro platinum (II) complex of formula II

   (aₘ)₂ Pt Cl₂ (II)

   wherein aₘ is as above defined,
   with a molar excess of an amine or a salt thereof of formula III

   H₂N-R (HCl)ₙ (H₂O)ₘ (III)

   wherein R, m and n are as above defined;
b) purification and optional crystallization of a so obtained compound of formula I.

The reaction is preferably carried out by using aqueous solutions or suspensions of the reactants, but, if desired, other solvents, such as C₁-C₃ alcohols, dimethylformamide and aqueous mixtures thereof, may be used. Particularly preferred reaction conditions are those providing the use of aqueous solutions containing the hydrochlorides of the amines of formula III in molar excess, which, with respect to cis-dichloro platinum (II) complex of formula II, can range from 1.5 to 5 molar equivalents. Particularly preferred molar excess ranges from 2.8 to 3.2 molar equivalents; best reaction conditions use at least three amine (or a salt thereof) molar equivalents per mole of dichloro platinum (II) complex of formula II.

The reaction may be carried out both by mixing the aqueous solutions of the reactant and by adding a dichloro platinum (II) complex of formula II to an aqueous solution of an amine salt of formula III: the mixture is then kept under stirring for a time ranging from some hours to some days, at a temperature ranging from room temperature to the solvent reflux one. Preferred reaction temperatures are those between 45 and 80°C, for periods of time from 24 to 56 hours. Particularly preferred reaction conditions are those comprising a reaction temperature of about 55°C and a period of time of about 48 hours.

The isolation and purification of the reaction products can be performed by column chromatography. For example, the reaction mixture, after concentrating it to small volume, can directly be purified by means of preparative chromatography in a "low pressure preparative TC system", using water as the eluant at a flow rate of 1.6 ml/min.

Such an operation is carried out on a chromatographic device supplied by Pharmacia LKB and consisting of: one peristaltic pump Varioperpex II 2120, one column KX (72 x 1.6 cm), having Sephadex G10 as stationary phase, one UV-Vis detector Uvicord S 2138, operating at λ 206 nm (cell lenght = 5 cm, ABS range = 1), one potentiometric recorder LKB 2210 and one fraction collector LKB 2111 Multirac.

The eluate is evaporated to dryness and, if desired, the solid residue is crystallized from an appropriate solvent. Preferred crystallization solvents are acetone, C₁-C₃ alcohols, diethyl ether, methyl or ethyl formate or acetate, acetonitrile and mixtures thereof.

Solvent elimination or concentration, if desired, is carried out by vacuum-evaporation or freeze-drying. The freeze-drying process is particularly preferred in the case of aqueous solutions.

Cis-triamino platinum (II) complexes of the invention of formula I are highly soluble salts into aqueous solvents.

The good to optimum water solubility of the "in toto" molecule is due to the platinum (II) complex part, which is present as salt countercation. In the compounds of formula I of the invention, wherein n is the integer 1, the contemporaneous solvation of the trialkylammonium group brings a further contribution to the solubility of the compounds into the aqueous solvents.

The dichloro platinum (II) complexes of formula II are well-known compounds, which are commercially available or easily prepared by means of well-known methods.

The amines (and the hydrochlorides thereof) of formula III, wherein X is O or NH, are esters or amides of 4-aminobenzoic acid and are well-known for their use in therapy as local anaesthetics and antiarhytmic agents. On their turn, the compounds of formula III, wherein X is CH₂, are Mannich bases of 4-aminoacetoophenone, and also they are well-known or can be prepared with well-known methods.

The formation of a cis-triamino chloro platinum (II) ionic complex of formula I, after reacting a cis-diamino platinum complex of formula II and an amine (or a salt thereof) of formula III, is rather unexpected, when instead in the same experimental conditions, the alternative use of 4-aminobenzoic acid (or the carboxylate salts thereof) gives only cis-diamino platinum (II) complexes having classical structure (Italian Patent Application 91A002072, of 26.07.1991).

As far as a wide literature describes the preparation of many cis-triamino-complexes (see references 13-16 in H. Steven Hollis et al., Journal of Medicinal Chemistry, 32, 128, 1989), only recently and in this reference, the preparation and pharmacological characteristics of 34 triamino-platinum complexes of formula IV

[Pt(NH₃)₂(N-donor)Cl]⁺ (IV)

wherein the "donor" group represents groups having different electron donor strenghth, such as alkylamines, pyrimidines, purines and pyridines, in the form of cis and trans analogues, have been described. Although all these triamino platinum complexes resulted at the screening less potent and active than cis platinum, the best combination of antitumor activity and potency was found in the compounds of formula IV having pyridine, 4-bromopyridine and 4-methylpyridine as N-donors. It was also observed that, having the same substituents, the analogues containing a secondary or tertiary heterocyclic ligand resulted active, on the contrary the activity was lost in the tris-amino platinum (II) complexes wherein a primary amino group (such as 2-propylamine, octilamine and N₂-1-methoxyisopropylamine) was the N-donor group. Generally, the cis configuration of platinum (II) complex proved to be an indispensable feature for the activity, as much as the nature of the chelating amine (Aₘ)₂ (compounds wherein (Aₘ)₂ is 1,2-diaminoethane or DACH were found inactive).

As far as known to date, the great part of the active cis-platinum analogues are neutral complexes capable of binding in bidentate way to their supposed biological target (DNA). Only for a very limited number of ionic platinum complexes a in vivo antitumor activity has been described; the ionic complexes endowed with a certain in vivo antitumor activity are for the major part anionic complexes (see J.P. Marquet et al., J. Natl. Cancer Inst., 70, 899, 1983 and E. Rerganetti et al., Inorg. Chim. Acta, 93, 167, 1984). The few examples of cationic complexes containing three amino groups as donors ((M.J. Cleare et al., Bioinorg. Chem., 2, 187, 1973 and J.P. Marquet et al., vide supra), described before in the above cited Hollis's paper, were always and invariably found devoid of activity in screening on S180a and L 1210.

The compounds of the invention, and particularly cis-diamino-chloro-4-[diethylammoniumethoxycarbonylphenyl]amino-platinum (II)⁺ bis-chloride monohydrate (cis-DPR), resulted instead to be endowed with a significant cytotoxyc activity, at least comparable with the one of cis-platinum (cis-DDP), when tested in vitro against P388 murine leukemic cells, K562 human erhytroleukemic cells and Jurkat human cells.

The compounds of the invention proved to be active also against cis-platin (cis-DDP) resistant tumor cells.

Cis-Dlatinum (cis-DDP) and cis-DPR cytotoxic activities have been evaluated on the sensible and cis-platinum resistant L 1210 murine leukemia cell lines, by using the 24 hours-thymidine uptake method, after continuous exposition to the drugs. In this test, L 1210/cis-DDP line proved to be about 11 times more resistant than the parental L 1210 line towards cis-DDP cytotoxic activity, while the above line showed a resistance index towards cis-DPR of about 1.9. As to the cis-DDP and cis-DPR IC₅₀s on L 1210/cis-DDP cell line, cis-DPR showed an activity about 10 times greater than the cis-DDP one. Cis-DPR resulted about 1.6 times more active than cis-DDP also on the parenteral L 1210 cell line. The results shown in Table 1 suggest that cis-DPR is capable of overcoming cisplatinum resistance.

**TABLE 1**

| | IC₅₀^{a} (µM) | |
|---|---|---|
| | cis-DDP | cis-DPR |
| L1210 | 0.84±0.09^{b} | 0.51±0.08^{c} |
| L1210/cis-DDP | 9.60±2.80 | 0.97±0.17^{c} |
| IR^{d} | 11.4 | 1.9 |

| | | |
|---|---|---|
| ^{a} Concentration producing 50% of inhibition | | |
| ^{b} Mean ±SD | | |
| ^{c} p<0,01 compared to cis-DDP | | |
| ^{d} Resistance index. | | |

On the other hand, and advantageously, the same compounds resulted significantly less active on blood peripheral mononuclear and non tumoral cells stimulated with phorbol acetate (cis-DPR: IC₅₀=1.88 ± 0.34(SD) versus cis-DDP: IC₅₀=0.97 ± 0,14(sD), p<0.05).

An in vivo compared antitumor activity assay in BDF1 mice, infected with P388 leukemic cells, showed the high activity of cis-DPR, at least comparable to the one obtained with the maximum tolerated dose of cis-DDP in terms of percent body weight variation (a 4.5% increase with cis-DPR versus a 10.6% decrease with cis-DDP) at the doses giving the highest increase in life span (ILS 75%, 21 and 8 mg/kg, respectively, after single administration).

The compounds of the invention are further characterized by their poor (almost absent) renal toxicity. For example, after acute intraperitoneal administration to female BDFl mice of equitoxic cis-DPR and cis-DDP doses (corresponding to the LD₅₀ of the two compounds, which were evaluated in 39.3 mg/kg (cis-DPR) and in 16.5 mg/kq (cis-DDP) dissolved into 0.9% aqueous NaCl), no evaluable kidney damages resulted after treatment with cis-DPR nor as plasma BUN increase, nor after examination of renal tubules. The damages are well evident in the case of cis-DDP, where plasma BUN increase appears to be well related with the degenerative modifications of the proximal tubules, which are completely absent in the case of the compounds of the invention.

Accordingly, the compounds of formula I are particularly advantageous and useful for the treatment of platinum-sensitive tumors. The high solubility and stability in the common aqueous solvents make the intravenous administration easy, contrarily to the other platinum (II) complexes, which are poorly hydrosoluble. The low renal toxicity of the compounds of the invention lead to think that, during and after their administration, the occurrence to the prolonged hydration regimen of the patient (in order to prevent hypotetic damages towards the kidney) will be less frequent with evident benefit for the therapeutic regimen compliance.

The effective dosage of the compounds of the invention can be determined by an expert physician with well-known and conventional methods. The correlation between the doses used with animals of several species and sizes and the human ones (based on mg/m² of body surface) is described by Freirech, E.J., et al., in Cancer Chemother. Rep., 50, N. 4, 219-244, 1966.

However, doses usually from 1 to 1200 mg/kg of the complex will be administered with a variable posology depending on several factors which are well known to the expert physician.

The treatment regimen can appropriately be varied according to the kind of tumor to be treated and the patient's conditions. The compounds of the invention are preferably administered in the form of sterile aqueous solutions, preferably by intravenous or intraartherial route, although other administration routes may be appropriate in particular cases.

The pharmaceutical compositions useful for the parenteral administrations comprise sterile aqueous solutions or powders for the extemporary preparation of solutions, as well as oleous formulations for intramuscular or intraperitoneal administration.

Other useful pharmaceutical compositions can be syrups or similar liquid compositions, as well as solid compositions such as tablets, capsules or the like. The following example illustrates a typical preparation of the compounds of the invention.

### EXAMPLE

30 mg of cis-diamino dichloro platinum (II) were added to 30 ml of an aqueous solution of N-diethylammoniumethyl p-aminobenzoate chloride (82 mg) in a 1:3 molar ratio. The mixture was kept under constant stirring for 48 hours at 55°C, then the mixture was concentrated, under reduced pressure in an rotary evaporator, down to 2 ml. The residue was injected in a low pressure TC preparative system; the elution was carried out with water at a flow rate of 1.6 ml/min. 3.2 ml fractions were collected, the fractions 24-36, containing the complex, were gathered and evaporated to dryness under reduced pressure in a warm bath (40°C). The solid residue was dissolved in methanol (10 ml); after diluting the solution with diethyl ether a crystalline precipitate separated. The crystallization was almost complete in about 1 hour at room temperature; after filtration and washing of the solids with 1:1 methanol/diethyl ether, 25 mg of cis-diamino-chloro-4-[diethylammoniumethoxycarbonylphenyl]amino-platinum (II)⁺ bis-chloride monohydrate (cis-DPR) were collected, which after recrystallization from the same solvent showed m.p. 173-176°C with decomposition.

Elemental analysis C₁₃H₂₇Cl₃N₄Pt.H₂O found %: C 26,58; H 4,95; N 9,36; calc. %: C 26,43; H 4,95; N 9,48.

The following Table 2 shows the most significant U.V., I.R. and ¹HNMR spectroscopy absorptions.

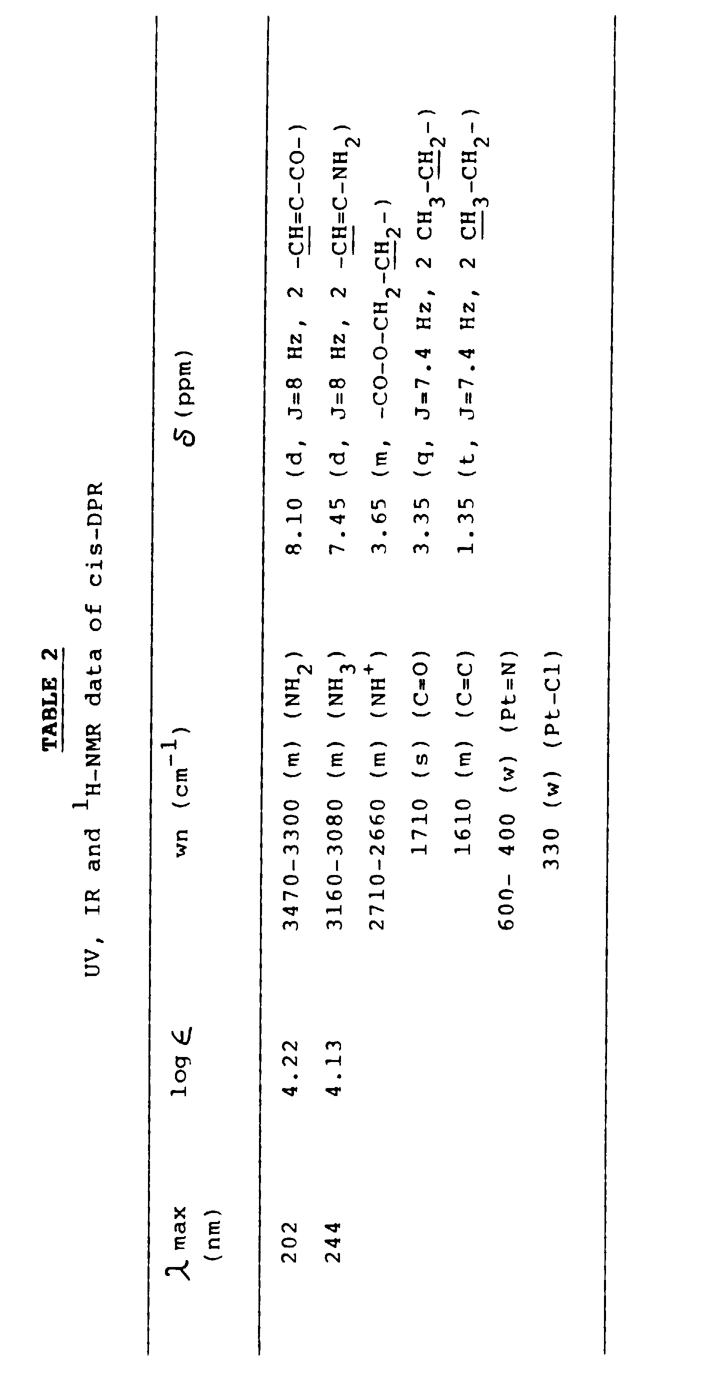

## Claims

1. Cis-triamino platinum (II) complexes or formula I
[(aₘ)₂ Pt H₂N-R Cl]⁺ Cl⁻ (HCl)ₙ (H₂O)ₘ (I)
wherein:
aₘ is an amino monodentate ligand selected from the group consisting of NH₃, straight or branched C₁-C₅ alkylamine, straight or branched C₁-C₃ dialkylamine, cyclopropylamine, cyclopentylamine, cyclohexylamine; or both aₘ groups, taken together, from a bidentate amino ligand selected from the group consisting of a C₂-C₈ alkyl-1,2-diamine or a straight or cyclic C₃-C₈ alkyl-1,3-diamine;
R is a group of formula p-NH₂-C₆H₄-CO-X-(CH₂)₂-NRaRb where X is selected from the group consisting of O, NH and CH₂, Ra and Rb, which are the same or different, are each a straight or branched C₁-C₄ alkyl and n and m, independently each other, are 0 or 1.

2. Compounds according to claim 1, wherein aₘ is a monodentate amine ligand selected from the group consisting of: NH₃, propylamine, isopropylamine, n-pentylamine, diisopropylamine, cyclopropylamine, cyclopentylamine and cyclohexylamine.

3. Compounds according to claim 1, wherein both aₘ together form a bidentate aminic ligand (aₘ)₂ selected from the group consisting of 1,2-ethylenediamine, 1,2-butanediamine, 1,2-cyclopentanediamine, 1,2-cyclohexanediamine, 1,2-cycloheDtanediamine, 1,3-propylenediamine, 1,1-bisaminomethylcyclopentane, 1,1-bisaminomethylcyclohexane.

4. Compounds according to claims 1-2, wherein both aₘ are NH₃, cis-oriented with respect to Pt atom.

5. Compounds according to claims 1-4, wherein X is O, Ra and Rb are both ethyl, m and n are the integer 1.

6. A compound according to claim 1, which is cis-diamino-chloro-4-[diethylammoniumethoxycarbonylphenyl]amino-platinum (II)⁺ bis-chloride monohydrate.

7. A process for the preparation of the compounds of the claims 1-6, which comprises:
a) reaction of a cis-diamino dichloro platinum (II) complex of formula II
(aₘ)₂ Pt Cl₂ (II)
wherein aₘ is as above defined,
with a molar excess of an amine or a salt thereof of formula III
H₂N-R (HCl)ₙ (H₂O)ₘ (III)
wherein R, m and n are as above defined;
b) purification and optional crystallization of a so obtained compound of formula I.

8. Pharmaceutical compositions containing one or more compounds of the claims 1-6 as active ingredients.

9. The use of the compounds of the claims 1-6 as therapeutic agents.

10. The use of the compounds of the claims 1-6 for the manufacturing of a medicament having antitumor activity.

## Patentansprüche

1. Cis-Triamino-Platin(II)-Komplexe der Formel I
[(aₘ)₂PtH₂N-R Cl]⁺Cl⁻(HCI)ₙ(H₂O)ₘ (I)
wobei:
aₘ ein einzähniger Amino-Ligand ist, der ausgewählt wird aus der aus NH₃, geradkettigen oder verzweigten C₁-C₅ Alkylaminen, geradkettigen oder verzweigten C₁-C₃ Dialkylaminen, Cyclopropylamin, Cyclopentylamin, Cyclohexylamin bestehenden Gruppe, oder beide aₘ-Gruppen zusammengenommen einen zweizähnigen Amino-Liganden bilden, der ausgewählt wird aus der aus einem C₂-C₅Alkyl-1,2-diamin oder einem geradkettigen oder cyclischen C₃-C₈Alkyl-1,3-diamin bestehenden Gruppe;
R eine Gruppe der Formel p-NH₂-C₆H₄-CO-X-(CH₂)₂-NRaRb ist, wobei X ausgewählt wird aus der aus O, NH und CH₂ bestehenden Gruppe, Ra und Rb, die gleich oder verschieden sind, jeweils geradkettiges oder verzweigtes C₁-C₄ Alkyl sind und n und m unabhängig voneinander O oder 1 sind.

2. Verbindungen nach Anspruch 1, wobei aₘ ein einzähniger Amin-Ligand ist, der ausgewählt wird aus der aus NH₃, Propylamin, Isopropylamin, n-Pentylamin, Diisopropylamin, Cyclopropylamin, Cyclopentylamin und Cyclohexylamin bestehenden Gruppe.

3. Verbindungen nach Anspruch 1, wobei beide aₘ gemeinsam einen zweizähnigen Amin-Liganden (aₘ)₂ bilden, der ausgewählt wird aus der aus 1,2-Ethylendiamin, 1,2-Butandiamin, 1,2-Cyclopentandiamin, 1,2-Cyclohexandiamin, 1,2-Cycloheptandiamin, 1,3-Propylendiamin, 1,1-Bisaminomethylcyclopentan, 1,1-Bisaminomethylcyclohexan bestehenden Gruppe.

4. Verbindungen nach Ansprüchen 1 bis 2, wobei beide aₘ NH₃ sind und bezogen auf das Pt-Atom cis-orientiert sind.

5. Verbindungen nach Ansprüchen 1 bis 4, wobei X O ist, Ra und Rb beide Ethyl sind, und m und n die ganze Zahl 1 sind.

6. Verbindung nach Anspruch 1, die cis-Diamino-chloro-4-[diethylammoniumethoxycarbonylphenyl]amino-platin(II)⁻-bis-chlorid-monohydrat ist.

7. Verfahren zur Herstellung der Verbindungen nach Ansprüchen 1 bis 6, welches umfaßt:
a) Reaktion eines cis-Diamino-dichloro-platin(II)-Komplexes der Formel II
(aₘ)₂PtCl₂
wobei aₘ wie oben definiert ist,
mit einem molaren Überschuß eines Amins der Formel III oder eines Salzes davon
H₂N-R(HCl)ₙ(H₂O)ₘ
wobei R, m und n wie oben definiert sind;
b) Reinigung und gegebenenfalls Kristallisation der so erhaltenen Verbindung der Formel I.

8. Pharmazeutische Zusammensetzungen, die eine oder mehr Verbindungen der Ansprüche 1 bis 6 als Wirkstoffe enthalten.

9. Verwendung der Verbindungen der Ansprüche 1 bis 6 als therapeutische Mittel.

10. Verwendung der Verbindungen nach Ansprüchen 1 bis 6 für die Herstellung eines Medikaments mit Antitumor-Aktivität.

## Revendications

1. Complexes de cis-triaminoplatine (II) de formule I
[(aₘ)₂ Pt H₂N-R Cl]⁺ Cl⁻ (HCl)ₙ (H₂O)ₘ (I)
dans laquelle:
aₘ est un ligand monodenté amino choisi parmi le groupe constitué du NH₃, d'une alkylamine en C₁-C₅ linéaire ou ramifiée, d'une dialkylamine en C₁-C₃ linéaire ou ramifiée, de la cyclopropylamine, de la cyclopentylamine, de la cyclohexylamine; ou les deux groupes aₘ, pris conjointement, forment un ligand amino bidenté choisi parmi le groupe constitué d'une alkyl-1,2-diamine en C₂-C₈ ou d'une alkyl-1,3-diamine en C₃-C₈ linéaire ou cyclique;
R est un groupe de formule p-NH₂-C₆H₄-CO-X- (CH₂)₂-NRaRb dans laquelle X est choisi parmi le groupe constitué de O, NH et CH₂, Ra et Rb, qui sont identiques ou différents, sont chacun un alkyle en C₁-C₄ linéaire ou ramifié et n et m, indépendamment l'un de l'autre, valent 0 ou 1.

2. Composés selon la revendication 1, dans lesquels aₘ est un ligand amine monodenté choisi parmi le groupe constitué de: NH₃, la propylamine, l'isopropylamine, la n-pentylamine, la diisopropyl-amine, la cyclopropylamine, la cyclopentylamine et la cyclohexylamine.

3. Composés selon la revendication 1, dans lesquels les deux aₘ forment conjointement un ligand aminé bidenté (aₘ)₂ choisi parmi le groupe constitué de la 1,2-éthylènediamine, la 1,2-butanediamine, la 1,2-cyclopentanediamine, la 1,2-cyclohexanediamine, la 1,2-cycloheptanediamine, la 1,3-propylènediamine, le 1,1-bisaminométhylcyclopentane, le 1,1-bisaminométhyl-cyclohexane.

4. Composés selon les revendications 1-2, dans lesquels les deux aₘ sont NH₃, orientés en cis par rapport à l'atome de Pt.

5. Composés selon les revendications 1-4, dans lesquels X est O, Ra et Rb sont tous les deux un éthyle, m et n sont l'entier 1.

6. Composé selon la revendication 1, qui est le bis-chlorure de cis-diamino-chloro-4-[diéthylammonium-éthoxycarbonylphényl]amino-platine (II)⁺ monohydraté.

7. Procédé de préparation des composés des revendications 1-6, qui comprend:
a) la réaction d'un complexe cis-diamino-dichloroplatine (II) de formule II
(aₘ)₂ Pt Cl₂ (II)
dans laquelle aₘ est comme défini ci-dessus,
avec un excès molaire d'une amine ou d'un sel de celle-ci de formule III
H₂N-R (HCl)ₙ (H₂O)ₘ (III)
dans laquelle R, m et n sont comme définis ci-dessus;
b) la purification et la cristallisation facultatives d'un composé ainsi obtenu de formule I.

8. Compositions pharmaceutiques contenant un ou plusieurs composés des revendications 1-6 à titre d'ingrédients actifs.

9. Utilisation des composés des revendications 1-6 à titre d'agents thérapeutiques.

10. Utilisation des composés des revendications 1-6 pour la fabrication d'un médicament ayant une activité antitumorale.
